# EUROPEAN PATENT APPLICATION

(11) **EP 2 039 236 A1**
(43) Date of publication of application: **25.03.2009**
(21) Application number: 07116877.7
(22) Date of filing: 20.09.2007
(51) Int. Cl.: A01D 44/00, A01G 33/00

(54) **Method for harvesting algae or plants and device used thereby**

(71) Applicant: Sbae Industries NV, 9950 Waarschoot (BE)
(72) Inventor: Vanhoutte, Koenraad, 9040 St Amandsberg (BE); Vanhoutte, Jan, 8750 Zwevezele (BE)
(74) Representative: Moens, Marnix Karel Christiane

(57) **Abstract**

Method for harvesting algae and/or plants in an open and continuous system whereby the algae and/or plants are grown on a submersed substratum, characterized in that the substratum is moved during growing of the algae or plants.

## Description

### Field of the Invention:

The present invention concerns a method for harvesting algae and/or plants.

### Background of the Invention:

It is generally accepted that cultivated algae or aqueous plants have a broad field of application. Algae are indeed an upcoming source of food and feed but are also becoming a major source of bio-energy.

Therefore there is a rising need for an efficient and economically acceptable method for growing algae or plants. One such method is described in WO 98/18344 and consists of providing a substratum in the form of a large net submersed in a water reservoir. The net is artificially inoculated with algae and the reservoir is filled with water and nutrients for the growing algae to a level wherein the substratum is just submersed but still able to capture sufficient sunlight necessary for algal growth. Once the substratum is fully overgrown with algae, harvesting can take place.

A drawback of this method is however that it necessitates vast areas of land to grow the algae. This not only increases capital investment costs for growing the algae, it also causes difficulties with respect to control of the growing process, such as nutrient renewal and temperature control.

A further drawback of the method is that the harvesting is a real bottle-neck for mass production, since the algae form a bio-film on the substrate which is hard to remove, and special equipment needs to be driven over the net to harvest the algae.

The goal of the present invention is to overcome the above mentioned drawbacks and provide for an efficient and economically sound harvesting method for algae and/or plants.

### Summary of the Invention:

The present invention concerns a method for harvesting algae or plants in an open and continuous system whereby the algae and/or plants are grown on a submersed substratum, characterized in that the substratum is moved during growing of the algae or plants.

### Detailed description of the Invention:

In accordance with the present invention the biomass that is grown or cultured which consists predominantly of micro-organisms. For the purpose of this invention it is understood that micro-organisms are all organisms, both single-celled and multicellular organisms, of which the largest dimension is smaller than 2 mm. However, natural microbial communities typically harbor many organisms of which the size dimensions clearly exceed the 2 mm criteria, e.g. filamentous algae, nematodes. Therefore and for the purpose of this invention we explicitly state that the term 'microbial communities' is understood to include all larger organisms that naturally occur in or around these communities. This includes but is not limited to for instance filamentous algae, nematode worms, crustaceans, insects etc.

The biomass grown in accordance with the present invention comprises microbial communities in general and of certain groups of micro-organisms in particular. The determination of a 'group' can be based on a taxonomical, ecological or any other functional classification.

One possible preferred example of a biomass produced by this invention is a biomass consisting of attached microbial communities and dominated by the algal group Bacillariophyta or 'diatoms'.

Further it is noted that for the present invention the term biomass also covers aqueous plants, preferably macro algae.

The present invention concerns a method for harvesting algae or plants in an open and continuous system whereby the algae and/or plants are grown on a submersed substratum, characterized in that the substratum is moved during growing of the algae or plants,

Preferably, the substratum is moved from an entry point to an exit point.

According to a preferred method of the invention, the algae or plants are submersed during movement from the entry point to the exit point.

According to a highly preferred embodiment, the substratum is inoculated at the entry point and/or the biomass is harvested at the exit point.

Preferably, the substratum is submersed in a water stream, the water streaming counter current with respect to the substratum.

According to the method of the invention it is further preferred that the substratum defines a main surface positioned askew and preferably perpendicular with respect to the water stream.

According to a preferred configuration of the present invention, the method includes creating waves within a water stream.

According to another preferred configuration of the present invention, the method includes positioning such as tilting the substratum in function of a light source.

According to another embodiment of the present invention also relates to a facility for harvesting algae or plants, the facility comprising (i) at least one waterway; (ii) means for creating a water stream therein; (iii) at least one substratum positioned in the waterway for growing algae or plants thereon (iv) harvesting means for removing the algae or plants from the substratum, characterized in that the facility further comprises (v) means for moving said substratum during growth of the algae or plants.

It is preferred that the facility further comprises means for positioning such as tilting the substratum in the waterway.

In accordance with the present invention, the movement of the substratum from a entry point (entrance) to an exit point during growth of the algae, induces a continuous growing and harvesting process, which in turn, allows an increased yield/m² with respect to traditional methods. As a result of the method of the present invention, this does not only facilitates control of the growing process but also allows a serious reduction in capital investment.

Another major advantage of the method according to present invention is that harvesting is performed at a fixed location, such that no complex mobile harvesting machinery is necessitated.

In accordance with one embodiment of the present invention, preferably the algae or plants are harvested when submersed. By harvesting the algae or plants when submersed, breakdown of the algae or plants is minimized and fall back into the water due to gravity is prevented, hence preventing losses of biomass. Additionally, the biomass upon exiting the water may dehydrate and/or decompose (at a cellular level) or eg. oxidise (at a molecular level). In accordance with the present invention, the biomass dehydration and degeneration is minimized. As a result, consistent and high quality biomass is obtained.

In accordance to a preferred embodiment, the substratum is submersed in a water stream, the water streaming counter current with respect to the movement of the substratum.

In accordance with the present invention, it has now surprisingly been found that the counter current system allows providing high nutrient levels for the highest density in biomass at the end of the growing process, while the lowest nutrient levels replenish the lowest densities of biomass at the initial colonization phase. Without wishing to be bound by any theory, it is believed that this configuration substantially prevents nutrient limitation to affect growth of biomass at any of the development phases of the microbial biomass. Additionally, the counter current stream allows preventing high nutrient levels at the end of the water stream, thereby limiting the need of purification of waste water.

Providing a counter current has some important advantages with respect to, especially, nutrient renewal and auto-inoculation.

With respect to auto-inoculation, it is to be noted that the configuration of moving substrata could be referred as a train of substrata. This train is in continuous motion. With a counter current system, a species rain will occur consisting of biomass suspended in the water stream. As the species rain moves downstream (or up to the substratum train) it encounters increasingly less colonized substrata which increases the probability that the new species can settle successfully and colonize these substrata.

Yet another preferred configuration of the method according to the invention includes the creation of waves in the water stream.

In accordance with the present invention, it has been found that a wave-action results in better capture of incident sunlight. Consequently, the entire configuration enables more light energy to enter the water column and subsequently be absorbed into microbial biomass. In addition the wave effect results in an alternating (so called 'flashing') light effect which has a beneficial effect on the growth of the biomass. The light effect results in a higher biomass of a selected composition.

In a highly preferred method of the present invention the method includes positioning such as tilting the substratum in function of a light source such as the sun.

As in the course of the day and/or with the changing of the seasons the position of the sun relative to the location of the substrata changes it would be beneficial if the inclination of the position of the substrata could be synchronized and/or altered in situ. This substrate positioning such as adaptable inclination optimizes the correlation between the incident sunlight versus the biomass growth. If there is too much sunlight (eg sunny summer days), the changed inclination can be made such as to increase shading which, in turn, helps to protect the biomass from overexposure leading to photo-inhibition. Whereas if there is too little sunlight (e.g. cloudy winter days) the inclination could be changed so as to achieve maximal capture of the perpendicular incident sunlight. Additionally, the adapted inclination could be further determined such as to maximize promotion of the growth and yield of biomass for a given species composition. The degree of inclination of the substrata may also depend on a given species as a particular community may prefer a more shaded habitat.

A highly preferred embodiment of the present invention is a method incorporating both creation of waves and substrata position inclination, thereby maximizing biomass growth. In particular, the inclination can be simultaneously optimally adapted to the incident angle of sunlight and the slopes of the waves on the water surface.

The present invention also concerns a facility for harvesting algae or plants in a open continuous system, the facility comprising (i) a waterway; (ii) means for creating a water stream therein; (iii) at least one substratum positioned in the waterway for growing algae or plants thereon (iv) harvesting means for removing the algae or plants from the substratum, characterized in that the system further comprises (v) means for moving said substratum from a entry point to an exit point of the waterway during growth of the algae or plants.

### Drawings and Examples:

The following preferred embodiments of a continuous open system and method according to the invention for growing algae or plants are herewith described in detail with reference to the accompanying drawings, in which:
- Figure 1: represents a algae harvesting facility according to the present invention;
- Figure 2: on a larger scale represents a partial cross section of figure 1 according to line II-II;
- Figures 3 to 5: represent a similar cross section according to lines III-III, IV-IV and V-V in figure 1;
- Figure 6: represents an alternative embodiment of figure 1;
- Figures 7 and 8: schematically represent examples of substrata that may be used for harvesting algae with a method according the present invention;

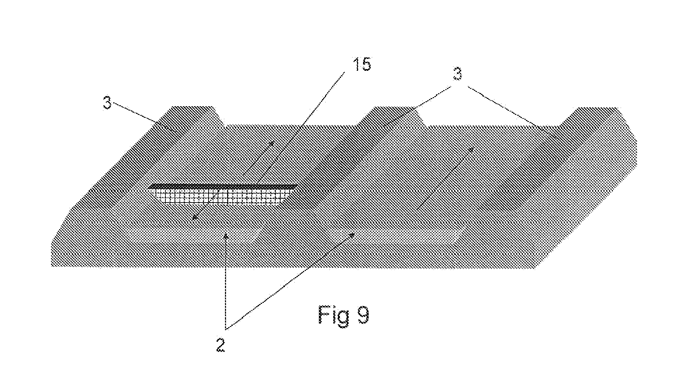

- Figure 9: schematically represents a moving substratum in a waterway;
- Figure 10: schematically represents a principle of tilting substrata.

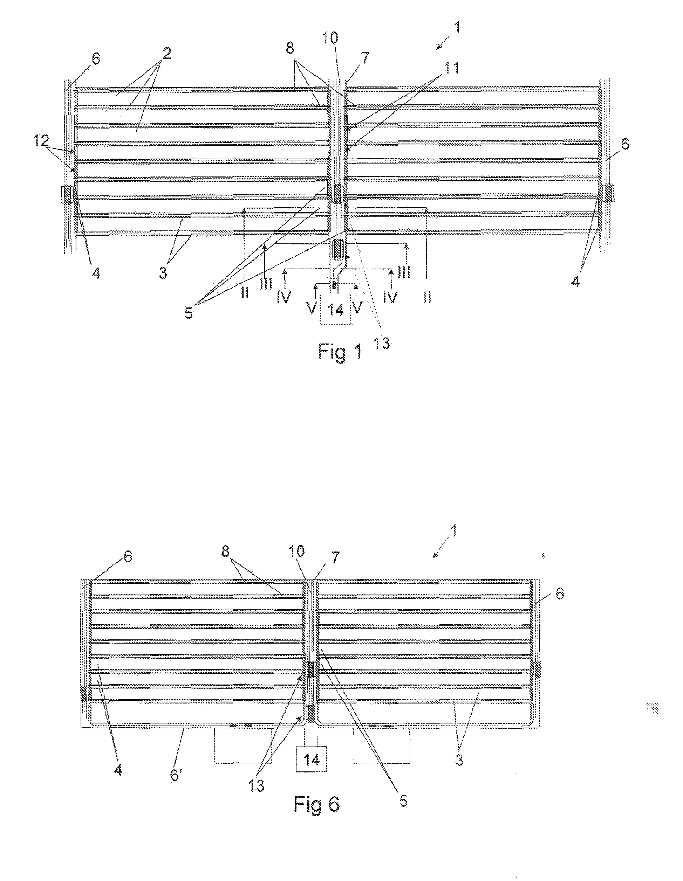

Figure 1 schematically represents a facility 1 for growing and harvesting algae by means of a method according the invention. The facility 1 comprises at least one and in this case two series of waterways 2. Each series comprises a number of parallel, adjacent waterways 2 that are spaced apart by ridges 3.

The waterways, which in this embodiment are linear, all comprise an entry point 4 and an exit point 5, whereby the entry points of the different waterways in one series are all in communication with a loading channel 6, whereas the exit points 5 of the different waterways of one series are in communication with what is further identified as a transport channel 7.

The facility preferably comprises two series of waterways, whereby the waterways of the second series are situated in the extension of the waterways of the first series. In this configuration it is preferred that both series have a common, central transportation channel, whereas the loading channels are situated at the opposed sides of the waterways.

Along the ridges 3 of the different channels are provided transport means 8 that may comprise gear rails, or a driving chain that are not represented in the drawings.

On the ridges are provided a number of carriers 9 comprising gear wheels that work in communication with the above gear rails, while in the loading channel 6 is provided a loading device for loading carriers on the gear rails.

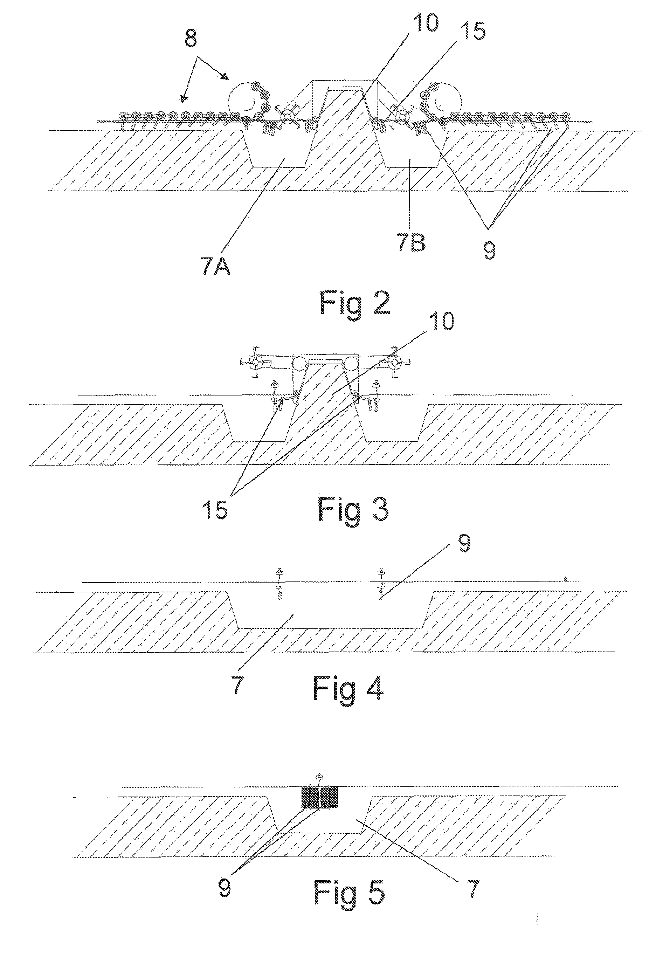

As represented in figure 2 the transport channel 7 is divided in two sub channels 7A-7B, segregated by a ridge 10, each sub channel being in communication with one series of waterways 2. Optionally, the transport channel houses buffer positions 11 for the carriers next to the exit point 5 of each waterway 2, similarly a buffer position 12 may be provided next to each waterway entry point in the loading channels 6.

A reloading channel 6' (figure 6) may also be provided for allowing recirculation of the carriers 9 and/or allowing recirculation of water from the exit point of the waterways to their entry points. This recirculation channel connects both the transport channel 7 and the loading channels 6. When used for the recirculation of water, it is preferred that nutrient replenishing points are provided in the recirculation channel or at the entry points of the waterways.

On the ridge 10 is provided a transportation device 13 allowing transporting the carriers 9 to a central harvesting unit 14. This harvesting unit 14 preferably comprises harvesting means allowing the removal of the biomass from the overgrown substrata. Such harvesting means may for example comprise a scraper. In this respect it is stressed that under harvesting should be understood each movement and process applied to the substratum from the exit point of the waterways up to the effective removal of the biomass from the substrata.

In the represented embodiment (figure 2) the transportation device 14 has two arms, one reaching to each subchannel 7A-7B, each arm being provided with a rotary disc with several hook shaped extensions that allow to lift carriers from the buffer position and place them on a collector rack 15 that can be moved along the transport channel to the harvesting unit 14 as represented in figures 3 to 5.

In at least one waterway 2, attached to a carrier 9 in a releasable manner, is provided a substratum 16 for growing algae. In an operation mode, the waterways 2 are filled with water to a level such that the substratum 16 or substrata positioned therein are entirely submersed.

The substratum 16 is preferably an artificial substratum in the form of for example a screen forming a main attachment surface, whereby the main attachment surface is oriented perpendicular or under a certain angle (askew) with respect to the moving direction or longitudinal direction of the waterway.

It is clear that preferably several carriers with substrata are positioned one after the other in the different waterways 2. This configuration of moving carriers with artificial substrata could be referred to as a train of carriers 9.

With respect to the artificial substratum it is remarked that, ideally, the artificial substrata 16 have a maximal attachment surface for a given volume by being fractal or fractal-like in shape and form without becoming so dissimilar from the natural substratum that the microbial communities no longer attach to them.

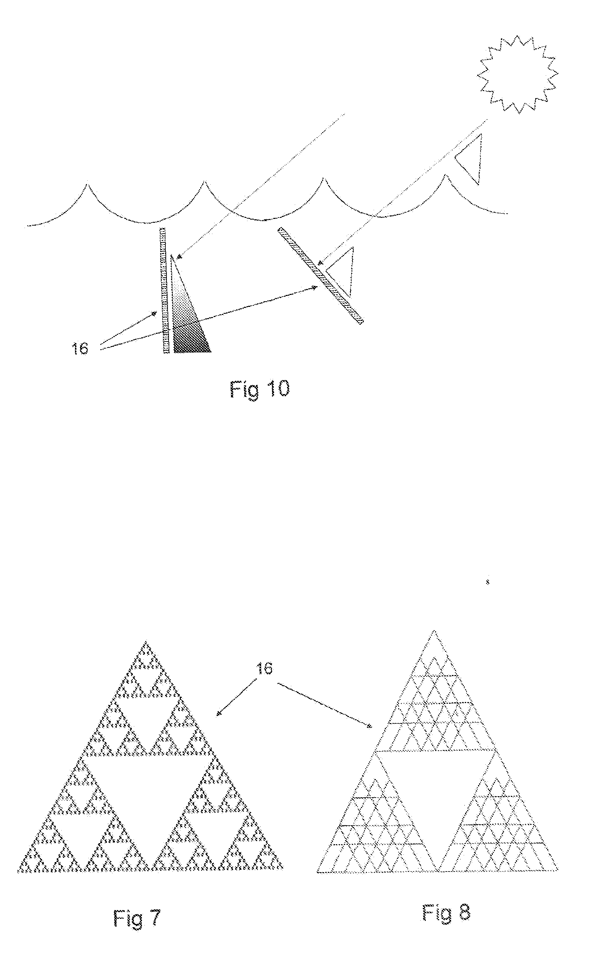

The substratum 16 is placed within the waterway 2, in such a way that when moved through the waterway 2, water flows over, across and through the substratum allowing for a transversal configuration of the system. The key problem of traditionally used substrata is that as biomass accrues on the substrata the system gets clogged and the flow is stopped, even up to a stage where the water passage is completely blocked. The flow is maintained since the fractal like nature of our substrata allows for the biomass to accrue on parts of the substrata whereas there are also wider openings that allow the flow to continue towards the next parts of the substrata. An example of execution is a carrier which holds a series of screens which form the substratum. These screens are perforated in a fractal patterns (for example Serpienski Gasket). This pattern divides a triangle in four equal triangle. The central triangle is open, while the three outer are again perforated in the self-similar fractal pattern. This is repeated infinitesimally in theory. The biomass will start to settle in the smallest perforated zones which will clog up gradually, later the larger perforated zones will clog, but there will always be a central opening which will allow the water flow to continue. At a given point the surface will be clogged to a certain degree and there will be a drag on the screen caused by the flow. This drag can be measured by a device and can be used as an indicator for the ideal moment of harvesting. The carrier with substrata is then extracted by the extractor. This configuration of internal fractal patterning allows transversal positioning of the carriers with substrata. Fractal patterning other then on a triangular surface can equally be selected eg. square, hexagon etc. As in well understood in the art, the fractal patterns and forms can be exhibited on the outside (external) of a surface (eg. Koch Curve). Another configuration would be that the fractal patterning is distributed not on one single screen (or in one plane) but is continued over a number of sequential screens. This would in principle be a '3 D fractaloid' or volumetric, whereas the above patterning on one screen is considered '2 D fractaloid' or planar. From the figures 7 and 8 the detailed fractal nature of the substrata and the transversal positioning which forces the water to flow through and across is further illustrated by a non-limiting example.

With respect to the water in the waterways and channels, it is remarked that the facility is preferably provided with means for creating a water stream running counter current in regard of the movement of the carriers and substrata through the waterways.

### The method for harvesting according to the invention is as follows:

In order to grow biomass, carriers 9 are loaded with substrata 16 and provided at a buffer position 12 at the entry point 4 of a waterway 2. From the buffer position 12 the carriers 9 are moved to the waterway 2 and further down this waterway by means of the transport means 8.

In general, the invention basically entails that the artificial substrata 16 are attached on moving carriers 9 in the waterway 2. These carriers 9 gradually move along the waterway 2 from an entry point 4 to an exit point 5. The water in the waterway preferably is running water that preferably streams from the exit point 5 to the entry point 4 (counter current).

The carriers 9 with artificial substrata 16 start at the entry point 4 as newly inserted artificial substrata on carriers 9 that have no or only very limited algae attached. As they move along the waterway 2 the artificial substrata 16 gradually become colonised with micro-organisms and a microbial biomass accrues. The biomass accrues during the entire period that the artificial substrata 16 on carriers 9 remain in this waterway 2.

Typically the accrued biomass becomes saturated after a certain period of time. Here this is defined in that it is saturated when it starts losing biomass because the biomass is being ripped of by the current (slouching).

When aiming to grow biomass at the most optimal productivity rather than at maximal achievable density, the period of time to saturation is consequently the period of residence of the carriers in the waterway 2 that is targeted in this moving configuration.

The desired period of residence (residence time) and the length of the waterways determine the speed with which the carriers move through the waterway. At the end of this period, as they arrive at the exit point 5 of the waterway 2, the artificial substrata 16 are therefore optimally overgrown with microbial communities. At this point they are discharged from the waterway 2 to the buffer position.

The substrata 16 are placed into this buffer position without the substrata 16 and the corresponding biomass being removed from the water. The buffer position is an aqueous environment. Removal of the substrata from the water would in principle be possible but this would result in partial or total rupture of the microbial communities through gravity, as a result of which a part or whole of the biomass would be lost in the stream. Moving the substrata into a buffer position without lifting them out of the water is more beneficial for the final yield of biomass per time unit per area unit.

From the buffer position, the substrata are transported through the transport channel 7 to the harvesting unit 14, where the biomass is removed from the substrata 16 and further subjected to a post treatment. As mentioned above, it is preferred that at least until harvesting, the biomass is maintained submersed. Therefore, obviously, the water level in the transport channel 7 and the corresponding buffer positions 11 should be sufficiently high to submerse the overgrown substrata 16.

Apart from the transport of the substrata, the transport channel 7 preferably has the function of entrance for the influent nutrient rich water that is directed through the waterways in counter current with respect to the moving substrata, although a separate channel or pipeline can be used to provide the nutrient rich influent water.

The buffer position in the transport channel is preferably constructed in such a way that a number of these artificial substrata can be placed there. The buffer position is continuously provided with new nutrient rich water (influent) as it is at the very beginning of each grow-out waterway 2. At this point the nutrient rich water contains its highest concentration of nutrients. This allows the biomass to reside for a period of time in situ without experiencing any adverse effects by stream reduction or nutrient limitation. This period of time makes it possible for the transportation device to move up and down the transversal channel without conflict.

The transportation of the substrata along the transport channel is achieved by means of the transportation device 13 collecting the biomass in such a way that they remain under optimal growth conditions (ie. submersed in the nutrient rich water stream). This configuration allows for maximal productivity and minimal loss of biomass. The transportation device 13 moves up and down the transport channel within a certain frame of time, enabled by the buffer positions 11. This time frame allows a steering system (eg. Computer, PLC) to determine the optimal sequence of collection. In principle this could be determined by observation and performed manually, but automatisation allows for a more optimal and economic use of the available resources (manpower, time, energy,...).

After collection the transportation device 13 moves along the transport channel 7 to a central harvesting and processing unit 14, where the substrata are delivered and the transportation device 13 can return to collect new overgrown substrata.

After removal of the biomass, the substrata 16 and carriers 9 can be reloaded in the waterways to maintain the train of carriers 9 running. This train is in continuous motion at a set speed. In this configuration of moving carriers it is clear that new carriers with artificial substrata must continuously be inserted at the beginning of each grow-out waterway. This is essential in order for the moving train of carriers to be complete and provide a continuous supply of accrued microbial biomass at the end. It is at this point of reloading that there is the additional possibility of altering the spacing between the carriers i.e. further or closer apart in the moving train of carriers. The spacing may be altered for a number of reasons: changing water properties, seasonality, desired biomass,....

The reloading of the grow-out waterways can be performed in a number of ways. The new carriers could be inserted manually or automatically by a machine or device. They could be inserted from a land-based position or a water-based position.

Ideally the reloading is performed via an integrated configuration, through which multiple functions are fulfilled. One possible configuration is to integrate the function of effluent channel and loading channel 6 of the new carriers. This channel 6 collects the nutrient depleted water or effluent from each grow-out waterway. The channel 6 brings the effluent to some central location for further treatment, usage, recycling or discharge. Along this channel the above mentioned loading device moves up and down which inserts new carriers in each grow-out waterway 2. The loading device has collected the new carriers from some central location where they have been prepared and brings them to each grow-out waterway 2.

Possibly an intermediate loading buffer may be considered as intermediate waiting position before the carriers are moved into the actual moving carrier train. This could be considered as a third function of the reloading channel. Alternatively this loading buffer 12 could be integrated in each grow-out waterway.

An alternative embodiment could consist of a land based device that places the carriers on a 'dry' loading buffer. From this dry loading buffer they are subsequently released by the automated buffer construction and positioned in the aqueous environment into the moving carrier train. The effluent water could be captured in a separate channel for further treatment or disposal.

An advantage of the 'wet' loading buffer is increased efficiency of colonization. The buffer typically will hold a number of carriers with artificial substrata. These will typically be placed close together; this is substantially closer than the interspacing they have in the grow-out channel. As such these closely spaced carriers will act as a filter which will 'capture' small particles of biomass from the effluent. As such microbial species will more easily colonize these carriers that are 'waiting' in the loading buffer.

A further advantage of an automatically loaded loading buffer 12 is that the loading device can load carriers 9 that have different types of artificial substrata. This allows for a certain sequence in the artificial substrata to be created within the grow-out waterway.

This allows for a greater control of the water stream in the grow-out waterways. Increased control of the water stream, for instance by using particular sequences of artificial substrata (fractaloid), allows for a better capture of kinetic energy by the growing biomass as well as for a improved renewal and absorption of the necessary nutrients by the growing biomass.

### Counter current

The direction of the water stream in the waterways 2 is preferably counter-current. In the present case the counter current is achieved by using the transport channel 7 which conducts the fresh nutrient rich water (influent) to the various grow-out waterways 2 to replenish the nutrient supply to the growing biomass. The counter current system provides high nutrients for the highest density in biomass at the end of the grow out waterway, while the lowest nutrient levels replenish the lowest densities of biomass at the initial colonization phase.

This configuration substantially prevents nutrient limitation to affect growth of biomass at any of the developmental phases of the microbial biomass.

In the counter current configuration the artificial carriers may be inoculated by a continuous species rain which is directly derived from small biomass particles loosening from the substrata in the waterways. This auto inoculation is especially beneficial in combination with an open system. With 'open' is meant that the system and especially the substrata are open to the environment, such that an external species rain derived from the regional species pool can inoculate the substrata. This natural inoculation is the mechanism that enables microbial communities to select new species as Complex Adaptive System to form part of the adapted microbial communities. In this way the communities continuously adapt to a changing environment and an open continuous system is created, whereby open stands for open to the regional species pool and continuous stands for continuously adapting communities.

As mentioned above, the counter current system furthermore results in the regionally derived species rain to be modified in a beneficial way. At the same time that species from the external species rain are caught by the biomass at the entrance of the influent water, inevitably a certain number of micro-organisms will continuously be discharged by this biomass accrued on the substrata in the carrier train. As a result the number of propagules (or colonization units) is higher as more organisms become suspended in the stream. This accrued microbial biomass at the end of the water way is already optimally adapted to the environment and therefore species derived from this community are likely to be better fitted for colonization in the downstream environment.

This in effect modifies the species-rain in such a way that the species-rain contains species that have already been selected as being better suited for colonization. The probability of successful colonization of organisms in this species rain is therefore significantly higher. As the number of propagules is higher this configuration does not exclude adaptation of the communities to changing conditions by incorporating new species from the regional species pool. The continuous inoculation with species from the already adapted communities is referred to as auto-inoculation. The mechanism that enables auto-inoculation allows for a more stable and predictable composition of the accrued biomass and hence final production of biomass. It also allows for a more efficient colonization of newly inserted artificial carriers.

Using the so-called complex adaptive system (CAS) approach and translating the CAS principle into a practical workable engineered open and continuous system thereby creating a simulation of a natural environment (habitat) for growing micro-organisms. The natural microbial communities are allowed to dynamically adapt to a changing simulated environment, such that the communities composition changes autonomously and accordingly communities adapt themselves to the changing simulating environmental conditions. The autonomous adaptation and local interaction results in self-organization of the communities. As a result, an optimal habitat is created wherein natural and diverse microbial communities can autonomously react and adapt resulting in an optimized production of biomass. In contrast with the classic production of mono-culturing techniques, using the natural and divers communities allows for a more stable habitat, in turn resulting in an improved biomass production and thus an increased yield per area.

It is further remarked that the stream of influent water can be adapted or even be made variable in function of the place in the waterway. Indeed, providing a variable influent stream (whether in speed or nutrient level) may increase control over the growing conditions of the biomass.

As an example, the stream speed can be chosen low at the exit point 5 of the waterways 2, to limit slouching, whereas nearer to the entry point 4 the desired water speed may be higher to allow creation of a so called species rain.

### Wave induction

Apart from the advantages towards nutrient repartition and auto-inoculation, the counter current configuration also has the advantage that it may lead to wave creation. Of course, additional means for creating waves can be provided for in the waterways 2.

The wave action of the water surface may be the direct result of the counter current configuration. The carriers with the artificial substrata are relatively shallow positioned underneath the water surface. The counter-current encounters the artificial substrata and are forced through the fractaloid patterns of perforation. However, part of the kinetic power of the water stream will hit the top edge of the artificial substratum and will push part of the water over the substratum.

As the configuration of the system is of such a nature that this occurs just beneath the water surface this will result in a wave being create as water flows/jumps over the substratum. This occurs repeatedly each time the water current encounters the top edge of a substratum. The result is an envisaged pattern of wave action along the entire length of the grow-out water way. This is beneficial in that the obtained wave-action results in better capture of incident sunlight. Consequently, the entire configuration enables more light energy to enter the water column and subsequently be incorporated into microbial biomass. We use the interaction between the kinetic energy of the water current and the exact positioning of the substrata to more efficiently convert solar power into biomass.

In addition the wave effect results in a flashing light effect which has a beneficial effect on the growth of the biomass. The flashing light effect results in a higher biomass of a selected composition.

It is clear that for creating wave action the distance between following carriers in the carrier train is important, but that the wave action also depends on other parameters such as water stream velocity, depth of the substrata under the water surface, substrata design, design of the waterway,... It will therefore be appreciated that some straight forward experimenting with one or more of these parameters will result in wave creation, without leaving the scope of the invention.

Additional wave induction, other than that possibly resulting from the counter current, may be provided for by known conventional techniques such as pumping means.

### Substrate positioning

Further, and as schematically represented in figure 10, it is preferred that the facility is provided with means that allow positioning such as tilting of the substrata in function of a light source.

As in the course of the day and/or with the changing of the seasons the position of the sun relative to the position of the artificial substrata changes it would be beneficial if the inclination of the artificial substrata could be altered in situ. This adaptable inclination helps to optimize the relation between the incident sunlight or other light source versus the biomass growth.

If there is too much sunlight (eg sunny summer days) the changed inclination increases shading which helps protect the biomass from overexposure which might cause photo-inhibition. Whereas if there is too little sunlight (eg cloudy winter days) the inclination could be changed so as to achieve maximal captivation of the perpendicular incident sunlight.

The inclination of the artificial substrata can be altered in the course of the day, either manually or automatically through some feedback system linked to an optical measuring device.

Additionally, the adapted inclination could be further determined so as to maximally promote the growth of a biomass of a particular composition. For instance a particular community may prefer a more shaded habitat.

Another aspect of this adaptable inclination is that the inclination can be simultaneously optimally adapted to the incident angle of sunlight and the slopes of the waves on the water surface, this in order to maximize biomass growth.

It is remarked that both the wave action and the inclination of the substrata are not only beneficial for a method of growing algae or plants on moving substrata, but that, for the same reasons as mentioned above, also have a positive impact on growing algae on fixed substrata.

The present invention is by no means limited to the embodiment and method described, but that the harvesting of algae or plants according to the invention can be achieved according to numerous variants without leaving the scope of the invention.

## Claims

1. Method for harvesting algae and/or plants in an open and continuous system whereby the algae and/or plants are grown on a submersed substratum, **characterized in that** the substratum is moved during growing of the algae and/or plants.

2. Method according to claim 1, **characterized in that** the substratum is moved from an entry point to an exit point.

3. Method according to claim 1 or 2, **characterized in that** the algae or plants are submersed during movement from the entry point to the exit point.

4. Method according to any of the preceding claims, **characterized in that** the substratum is inoculated at the entry point

5. Method according to any of the preceding claims, **characterized in that** the algae or plants are harvested at the exit point.

6. Method according to any of the preceding claims, **characterized in that** the substratum is submersed in a water stream, the water streaming counter current with respect to the substratum.

7. Method according to claim 6, **characterized in that** the substratum defines a main surface positioned askew and preferably perpendicular with respect to the water stream.

8. Method according to any of the preceding claims, **characterized in that** waves are created within a water stream.

9. Method according to any of the preceding claims, **characterized in** positioning the substratum in function of a light source.

10. A facility for harvesting algae or plants, the facility comprising (i) at least one waterway; (ii) means for creating a water stream therein; (iii) at least one substratum positioned in the waterway for growing algae or plants thereon (iv) harvesting means for removing the algae or plants from the substratum, **characterized in that** the facility further comprises (v) means for moving said substratum during growth of the algae or plants.
